# EUROPEAN PATENT SPECIFICATION

(11) **EP 2 210 629 B1**
(45) Date of publication and mention of the grant of the patent: **06.01.2016**
(21) Application number: 10003381.0
(22) Date of filing: 14.02.2005
(51) Int. Cl.: A61M 1/36, A61M 1/02

(54) **Apparatus and methods for separating a volume of composite liquid into at least two components**
Vorrichtung und Verfahren zum Trennen von einem Flüssigkeitsgemischvolumen in wenigstens zwei Komponenten
Dispositif et procédé pour séparer un volume d'un liquide composite en au moins deux composants

(30) Priority: 20.02.2004 US 546637 P
(43) Date of publication of application: 28.07.2010
(62) Divisional of application: 05003022.0
(73) Proprietor: Terumo BCT, Inc., Lakewood, CO 80215 (US)
(72) Inventor: Holmes, Brian M., Evergreen, Colorado 80439 (US); Van Waeg, Geert, 1200 Brussels (SE); Pihlstedt, Peter, 11422 Stockholm (SE)
(74) Representative: Roberts, Mark Peter

(56) References cited:
- WO-A-00/54823
- WO-A-03/089027
- WO-A-2004/018021
- US-A- 5 733 545

## Description

The present invention relates to an apparatus for separating a volume of composite liquid into at least two components.

The apparatus of the invention is particularly appropriate for the separation of biological fluids comprising an aqueous component and one or more cellular components. For example, potential uses of the invention include: extracting a plasma component and a red blood cell component from a volume of filtered blood obtained by flowing of a volume of whole blood through a filter removing platelets and white blood cells therefrom; extracting a plasma component, a platelet component and a red blood cell component from a volume of filtered blood obtained by flowing a volume of whole blood through a filter removing white blood cells therefrom; extracting a plasma component an a cellular component (including platelets, white blood cells, and red blood cells) from a volume of whole blood, the cellular component being subsequently filtered so as to remove platelets and white blood cells therefrom; extracting a plasma component, a platelet component, and a red blood cell component from a volume of whole blood, the white blood cells being subsequently removed by filtration from the platelet component and the red blood cell component.

An apparatus for processing blood components is known from document WO 03/089027. This apparatus comprises a centrifuge adapted to cooperate with an annular separation bag connected to at least one product bag, e.g. a platelet component bag. The centrifuge includes:
- a rotor having a turntable for supporting the separation bag, and a central compartment for containing the product bag connected to the separation bag; and
- a squeezing system for squeezing the separation bag and causing the transfer of a separated component (e.g. platelets suspended in a diluting solution) from the separation bag into the product bag.

An object of the present invention is to design a centrifugation apparatus that can perform an optimized separation process for separating, in a minimum amount of time, a composite fluid, such as whole blood, into at least two high quality components.

The invention is defined in the claims.

The flow rate (or the various flow rates) at which each separated component is transferred into the corresponding component bag is selected so as:
- to be as high as possible while, at the same time,
- to be low enough for not causing any substantial contamination of the separated component with contiguous components (for example, the contamination of a plasma component with blood cells), and
- to be low enough for not having any substantially detrimental impact on the cells of which the separated component may be composed (for example, the activation of platelets, or the hemolysis of red blood cells).

One of the major interests of the invention is therefore to make it possible to separate a volume of composite liquid as quickly as possible into components of high quality (i.e. substantially not contaminated by other components and not damaged).

Other optional features of the apparatus according to the invention are as follows:
- The squeezing member is further for causing the transfer of the third component into a third component bag connected to the separation bag;
   - the memory is further for storing information related to at least one third transfer flow rate of the third component into the third component bag, whereby the at least one third transfer flow rate is different from the at least one second transfer flow rate; and
   - the control unit is further programmed:
      - for receiving from the memory the information related to the at least one third transfer flow rate; and
      - for causing the squeezing member to squeeze the separation bag so as to transfer the third component from the separation bag into the third component bag at the at least one third transfer flow rate.
- The at least one first transfer flow rate is a substantially constant flow rate.
- The at least one second transfer flow rate comprises an initial flow rate and a final flow rate, the final flow rate being lower than the initial flow rate.
- The at least one third transfer flow rate comprises an initial flow rate and a final flow rate, the final flow rate being lower than the initial flow rate.
- In a first variant of the invention, the control unit is further programmed:
   - for causing, upon sedimentation of the first, second, and third components in the separation bag, the squeezing member to squeeze the separation bag so as to transfer a first portion of the first component from the separation bag into the first component bag at the at least one first transfer flow rate, while a second portion of the first component remains in the separation bag; and
   - for causing, after the transfer of the first portion of the first component into the first component bag, a variation of the centrifugation speed so as to mix the second component with the second portion of the first component and form the intermediate component.
- In the first variant of the invention, the control unit is further programmed for
   - causing the rotor to rotate at a first centrifugation speed during the transfer of the first portion of the first component from the separation bag into the first component bag; and
   - causing a rapid decrease of the centrifugation speed from the first centrifugation speed to a second centrifugation speed so as to mix the second component with the second portion of the first component and form the intermediate component.
- Alternately, in a second variant of the invention, the control unit is further programmed:
   - for causing, upon sedimentation of the first, second and third components in the separation bag, the squeezing member to squeeze the separation bag so as to transfer a first portion of the first component from the separation bag into the first component bag at the at least one first transfer flow rate while a second portion of the first component remains in the separation bag;
   - for causing, after the transfer of the first portion of the first component into the first component bag, a rapid decrease in the centrifugation speed from a first centrifugation speed to a second centrifugation speed so as to cause a mixing of the second component with the second portion of the first component and the third component; and
   - for causing, after the mixing of the second component with the second portion of the first component and the third component, an increase in the centrifugation speed from the second centrifugation speed to a third centrifugation speed so as to separate the third component from an intermediate component comprising the second component and the second portion of the first component.
- The control unit is further programmed for causing a transfer of air from the separation bag into one of the component bags before the transfer of the first component from the separation bag into the first component bag.
- The apparatus further comprises:
   - a first valve member mounted on the rotor for interacting with a first tube connecting the separation bag to the first component bag and selectively allowing or blocking a flow of fluid therethrough;
   - a second valve member mounted on the rotor for interacting with a second tube connecting the separation bag to the intermediate component bag and selectively allowing or blocking a flow of fluid therethrough; and
   - a third valve member mounted on the rotor for interacting with a third tube connecting the separation bag to the third component bag and selectively allowing or blocking a flow of fluid therethrough,
      wherein the control unit is further programmed for controlling the first, the second and the third valve members.
- The apparatus further comprises:
   - a first sensor for detecting the third component on a pathway of a fluid to the intermediate component bag;
   - a second sensor for detecting the third component on a pathway of a fluid to the intermediate component bag upstream of the first sensor; and
   - a third sensor for detecting the third component on a pathway of a fluid to the first component bag.
- The control unit is further programmed for causing the transfer of the at least one portion of the first component from the separation bag into the first component bag by causing:
   - the first valve member to open;
   - the second and third valve members to close; and
   - the squeezing member to squeeze the separation bag until the third sensor detects the third component on a pathway of a fluid to the first component bag.
- The control unit is further programmed for causing the transfer of the intermediate component from the separation bag into the intermediate component bag by causing:
   - the second valve member to open;
   - the first and third valve members to close; and
   - the squeezing member to squeeze the separation bag until the first sensor detects the third component on a pathway of a fluid to the intermediate component bag.
- The control unit is further programmed for causing the transfer of the intermediate component at the initial flow rate until the second sensor detects the third component and at the final flow rate when the first sensor detects the third component.
- The control unit is further programmed for causing the transfer of the third component from the separation bag into the third component bag by causing:
   - the third valve member to open;
   - the first and the second valve members to close; and
   - the squeezing member to squeeze the separation bag until it is substantially empty.
- The first sensor is also adapted to detect a liquid on a pathway from the separation bag to the third component bag, and the control unit is further programmed for causing a transfer of air from the separation bag into the third component bags by causing:
   - the first and second valve members to close;
   - the third valve member to open; and
   - the squeezing member to squeeze the separation bag until the first sensor detects the first component.
- The rotor comprises:
   - a turntable for supporting the separation bag, and
   - a lid that can be secured to the turntable for enclosing the flexible separation bag, and
- the squeezing member comprises:
   - a flexible diaphragm secured to the turntable,
   - a pumping station for pumping a hydraulic fluid into and out of an expandable chamber delimited between the turntable and the flexible diaphragm, whereby the flexible separation bag is being squeezed against the lid when the hydraulic fluid is pumped into the expandable chamber; and
   - a pressure sensor for sensing the pressure of the hydraulic fluid and detecting when the separation bag is substantially empty.
- The control unit is further programmed for causing the transfer of the third component at a first flow rate until the hydraulic pressure measured by the pressure sensor reaches a determined pressure threshold, and at a second flow rate after the hydraulic pressure measured by the pressure sensor has reached the determined pressure threshold, the second flow rate being lower than the first flow rate.

Other features and advantages of the invention will appear from the following description and accompanying drawings, which are to be considered exemplary only.

In the accompanying drawings:
Figure 1 is a schematic view of set of separation and collection bags designed for cooperating with a separation apparatus according to the invention;
Figure 2 is a top plan view of a separation bag designed for cooperating with a separation apparatus according to the invention;
Figure 3 is a schematic view, partly in cross-section, of a separation apparatus according to the invention;
Figure 4 is a cross-section view of the rotor of a separation apparatus according to the invention; and
Figure 5 is a perspective view of the upper part of the rotor of the separation apparatus of figure 4.

For the sake of clarity, the invention will be described with respect to a specific use, namely the separation of whole blood into at least two components, in particular into a first component comprising plasma, a second component comprising platelets and a third component comprising red blood cells. It should be understood however that this specific use is exemplary only.

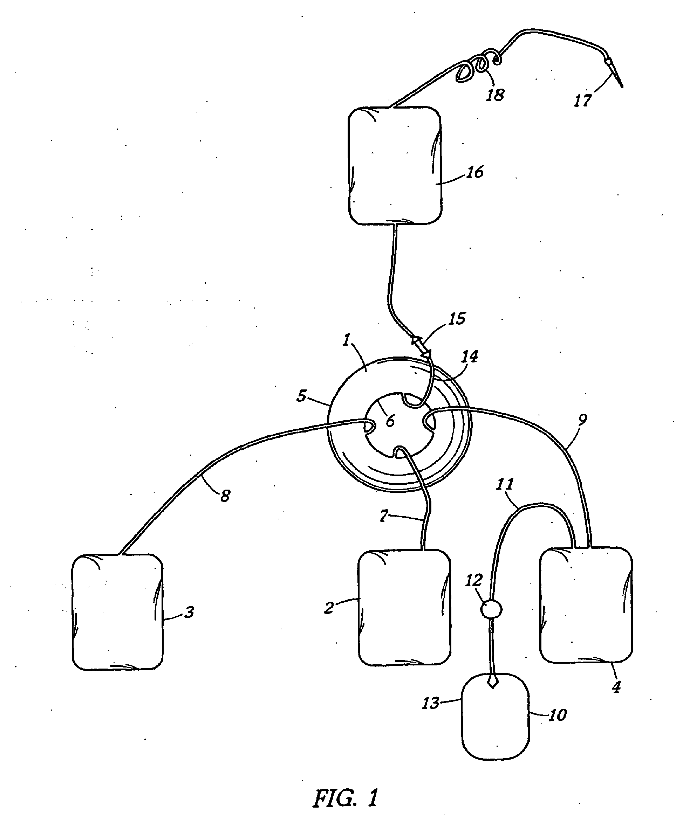

A set of separation bags adapted to the separation of whole blood into a plasma product, a platelet product and red blood cell product is shown in figure 1. This set comprises a separation bag 1 and three product bags 2, 3, 4. The separation bag 1 is annular and has an outer circular edge 5 and an inner circular edge 6. Variants of the separation bag include one or two radial walls extending from the inner edge 6 to the outer edge 5 so that the chamber defined within the bag, instead of being annular, has a C-shape with the C being more or less open. Also the separation bag can be shaped so as to fit either on a flat support surface or on a frusto-conical support surface of the rotor of a centrifuge. The first product bag 2, intended for containing the plasma product, is connected by a first tube 7 to the separation bag 1, at the inner edge 6 thereof. The second product bag 3, intended for containing the platelet product, is connected by a second tube 8 to the separation bag 1, at the inner edge 6 thereof. The third product bag 4, intended for containing the red blood cell product, is connected by a third tube 9 to the separation bag 1, at the inner edge 6 thereof. It is connected to a secondary bag 10 by a tube 11 having two segments respectively connected to the inlet and the outlet of a leukoreduction filter 12 (a filter for removing white blood cells). The secondary bag 10 contains a volume of storage solution for red blood cells. A plug 13 removable from within the secondary bag 10 (so-called "frangible pin", for example) blocks a liquid flow through the connecting tube 11 and prevents the storage solution from flowing into the third product bag 4. The bag set further comprises a supply tube 14 that is connected at one end to the separation bag 1, at the inner edge thereof. The other end of the supply tube 14 is either connected to a cannula, in which case the volume of blood to be separated is to be directly drawn from a donor into the separation bag 1, or connected directly or through a sterile connector 15 to a collection bag 16 connected in turn to a cannula 17 by a donor tube 18 (as shown in figure 1). The bag into which a volume of blood from a donor is to be directly transferred (the separation bag 1 or the collection bag 16) contains a volume of anti-coagulant solution (typically about 70 ml of a solution of sodium citrate for a blood donation of about 450 ml).

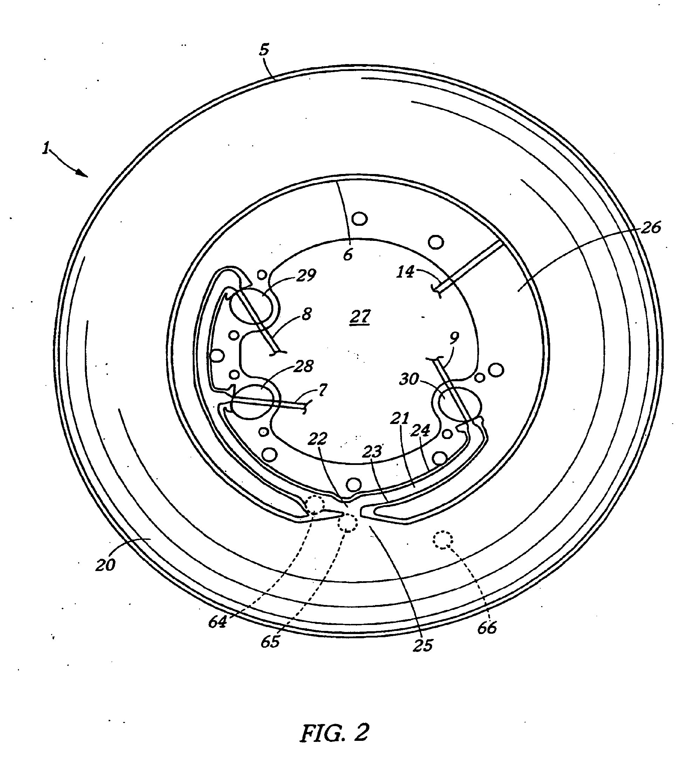

Figure 2 shows a separation bag 1, which is made of two superposed sheets of a flexible plastic material that are joined together by welded lines defining an annular chamber 20 communicating with an inner semi-circular distribution channel 21 via a narrow passage 22. More specifically, the annular chamber 20 is defined by a first circular welded line forming an outer edge 5, and a second circular welded line forming an inner edge 6, the two circular lines being substantially concentric. The distribution channel 21 is defined by two substantially parallel and semi-circular welded lines, forming an outer edge 23 and an inner edge 24 of the distribution channel 21. The inner edge 6 of the annular chamber and the outer edge 24 of the distribution channel 21 join in two points and define therebetween the passage 22. The inner edge 6 of the annular chamber 20 inwardly converges towards both junction points, and the resulting concavity in the otherwise circular inner edge 6 of the annular channel 20 defines a triangular bay area 25 in the annular chamber 20 just upstream of the passage 22.

The passage 22 opens in the distribution channel 21 at about two third of the length of the channel. With respect to the passage 22, the distribution channel 21 can therefore be defined as comprising a longer segment and a smaller segment that are interconnected and extend in opposite directions from the passage 22. The tube 9 connecting the product bag 4 for a red blood cell product to the separation chamber 1 is connected to the smaller segment of the channel 21, at the end thereof. The tube 8 connecting the product bag 3 for a platelet product to the separation chamber 1 is connected to the longer segment of the channel 21, at the end thereof. The tube 7 connecting the product bag 2 for a plasma product to the separation chamber 1 is connected to the longer segment of the channel 21, at about half of its length. The tube 14 for connecting a source of whole blood (donor or collection bag 16) to the separation bag 1 is connected to the annular chamber 1 at the inner edge 6 thereof, at about one third of the circumference of the inner edge 6 from the passage 22, in the same direction as the direction in which the small segment of the distribution channel 21 extends.

The distribution channel 21 and an end portion of the tubes 7, 8, 9, 14 are embedded in a disk-shaped support 26 made of a sheet of semi rigid plastic material, which is secured at its periphery to the inner edge 6 of the annular chamber 20. The disk-shaped support 26 comprises a large cut-out in the middle thereof, as well as three small circular cut-outs 28, 29, 30 adjacent to the connecting points of the tubes 7, 8, 9 to the distribution channel 21. The circular cut-outs 28, 29, 30 are positioned with respect to the end portion of tubes 7, 8, 9 so that each tube extends along a diameter of the corresponding circular cut-out and is therefore maintained straight over a portion of its length by the disk-shaped support 26.

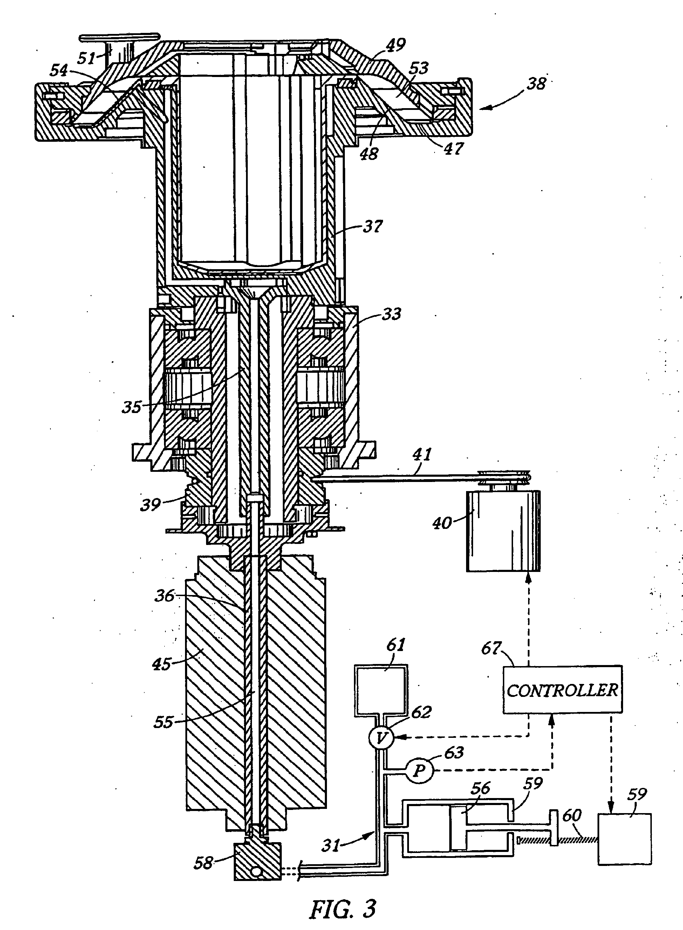

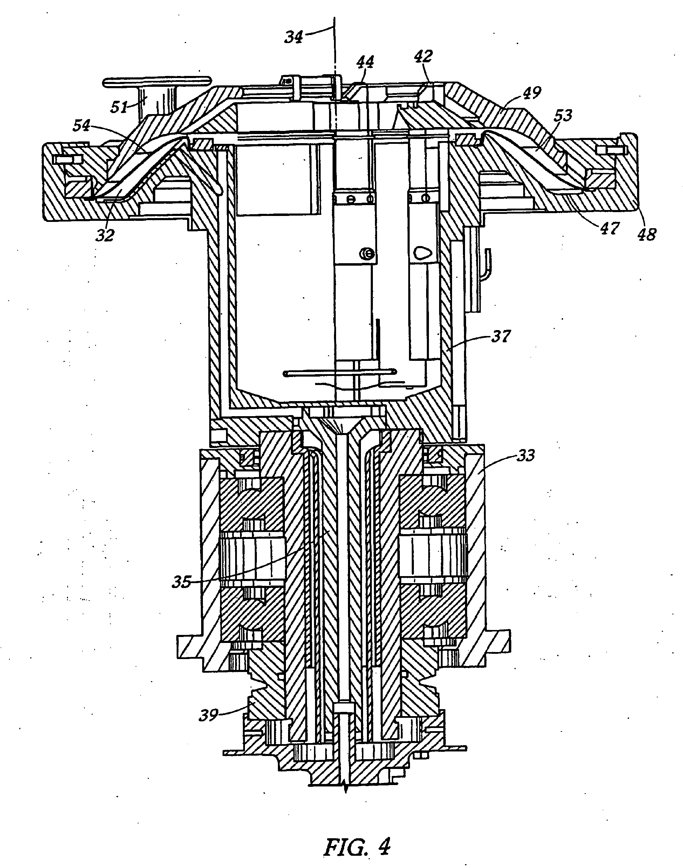

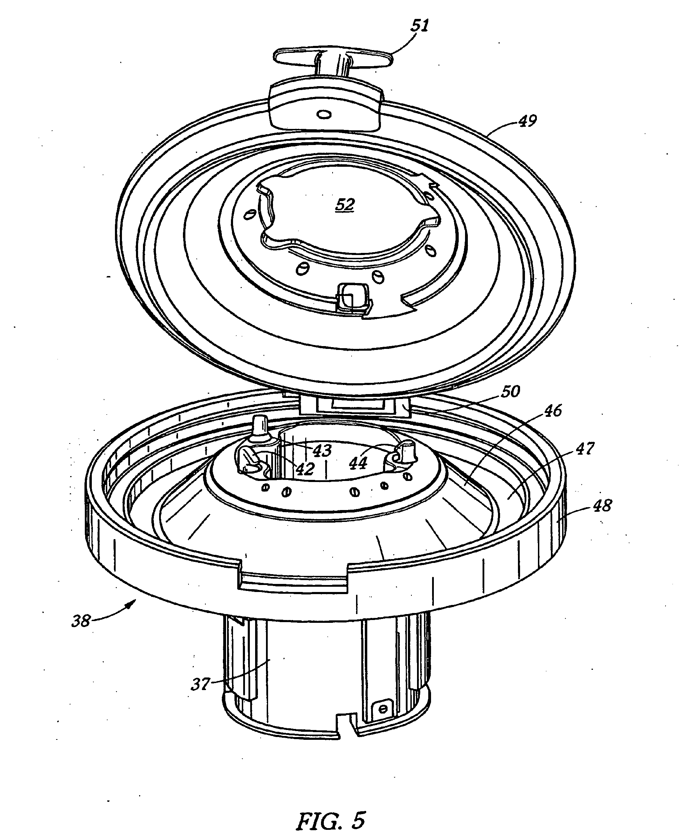

Figures 3, 4, 5 show an apparatus for separating a volume of composite liquid by centrifugation. The apparatus comprises a centrifuge adapted for receiving the set of separation and product bags shown in figures 1 and 2, and a squeezing system for squeezing the separation bag and causing the transfer of separated components into the product bags.

The centrifuge comprises a rotor that is supported by a bearing assembly 33 allowing the rotor to rotate about a vertical central axis 34. The rotor comprises a cylindrical rotor shaft 35, 36, a cylindrical container 37 that is connected to the rotor shaft 35, 36 at the upper end thereof so that the longitudinal axis of the rotor shaft 35, 36 and the longitudinal axis of the container 37 are aligned with the central axis 34 of the rotor, and a circular turntable 38 connected to the container 37 at the upper end thereof so that the central axis of the turntable 37 is aligned with the central axis 34 of the rotor. The rotor shaft comprises a first upper portion 35 and a second lower portion 36. The upper portion 35 of the shaft extends in part through the bearing assembly 33. A pulley 39 is connected to the lower end of the upper portion 35 of the shaft.

The centrifuge further comprises a motor 40 coupled to the rotor by a belt 41 engaged in a groove of the pulley 39 so as to rotate the rotor about the central vertical axis 34.

The separation apparatus further comprises three pinch valve members 42, 43, 44 that are mounted on the rotor for selectively blocking or allowing a flow of liquid through a plastic tube, and selectively sealing and cutting a plastic tube. Each valve 42, 43, 44 comprises an elongated cylindrical body and a head having a groove that is defined by a stationary upper jaw and a lower jaw movable between an open and a closed position, the groove being dimensioned so that one of the plastic tubes 7, 8, 9 of the bag set shown in figures 1 and 2 can be snuggly engaged therein when the lower jaw is in the open position. The elongated body contains a solenoid-actuated mechanism for moving the lower jaw and it is connected to a radio frequency generator providing the energy that is necessary for sealing and cutting a plastic tube. The pinch valve members 42, 43, 44 are mounted inside the cylindrical container 37, adjacent the interior surface thereof, so that their longitudinal axis is parallel to the central axis 34 of the rotor and their heads protrude above the rim of the container 37. The three circular cut-outs 28, 29, 30 of the support portion 26 of the separation bag 1 shown in figure 2 are so dimensioned and positioned as to allow for the engagement of the heads of the three pinch valve members 42, 43, 44 therethrough, with the portions of the tubes 7, 8, 9 extending across the circular cut-outs 28, 29, 30 oriented so as to face the groove in the heads of the pinch valve members 42, 43, 44. Electric power is supplied to the pinch valve members 42, 43, 44 through a slip ring 45 that is mounted around the lower portion 36 of the rotor shaft.

The turntable 38 comprises a central frusto-conical portion 46, the upper, smaller edge of which is connected to the rim of the container 37, an annular flat portion 47 connected to the lower, larger edge of the frusto-conical portion 46 and an outer cylindrical flange 48 extending upwards from the outer periphery of the annular portion 47. The turntable 38 further comprises a vaulted circular lid 49 that is secured to the flange 48 by a hinge 50 so as to pivot between an open and a closed position. The lid 49 is fitted with a lock 51 by which it can be blocked in the closed position. The lid 49 comprises a large cut-out 52 in its upper part that gives access to the cylindrical container 37 of the rotor. The lid 49 has an annular interior surface that is so shaped that, when the lid 49 is in the closed position, it defines with the frusto-conical portion 46 and the annular flat portion 47 of the turntable 38 a frusto-conical annular compartment 53 having a radial cross-section that has substantially the shape of a parallelogram. The frusto-conical annular compartment 53, later the "separation compartment", is intended for containing the separation chamber of the separation bag shown in figure 2.

The squeezing system for squeezing the separation bag within the separation compartment 53 and causing the transfer of separated components into the product bags comprises a flexible annular diaphragm 54 that is so shaped as to line the frusto-conical portion 46 and the annular flat portion 47 of the turntable 38, to which it is secured along its smaller and larger circular edges. The squeezing system further comprises a hydraulic pumping station 31 for pumping a hydraulic liquid in and out an expandable hydraulic chamber 32 defined between the flexible diaphragm 54 and the turntable 38, via a duct 55 extending through the rotor from the lower end of the lower portion 36 of the rotor shaft to the turntable 38. The pumping station 31 comprises a piston pump having a piston 56 movable in a hydraulic cylinder 57 fluidly connected via a rotary fluid coupling 58 to the rotor duct 55. The piston 56 is actuated by a stepper motor 59 that moves a lead screw 60 linked to the piston rod. The hydraulic cylinder 57 is also connected to a hydraulic liquid reservoir 61 having an access controlled by a valve 62 for selectively allowing the introduction or the withdrawal of hydraulic liquid into and from a hydraulic circuit including the hydraulic cylinder 57, the rotor duct 55 and the expandable hydraulic chamber 32. A pressure gauge 63 is connected to the hydraulic circuit for measuring the hydraulic pressure therein.

The separation apparatus further comprises three sensors 64, 65, 66 for detecting characteristics of the separation process occurring within a separation bag when the apparatus operates. The three sensors 64, 65, 66 are embedded in the lid 49 so as to face the separation bag 1 as shown in figure 2, when the lid 49 is closed. The first sensor 64 (later the "channel sensor") is embedded in the lid 49 so as to be positioned over the longer segment of the distribution channel 21. The channel sensor 64 is able to detect the presence of absence of liquid in the distribution channel 21 as well as to detect red blood cells in a liquid. The second sensor 65 (later the "bay sensor") is embedded in the lid 49 so as to be positioned over the bay area 25. The bay sensor 65 is able to detect red blood cells in a liquid. The third sensor 66 (later the "bag sensor") is embedded in the lid 49 so as to be positioned over the separation chamber 20, at about one third of the breadth of the separation chamber from the inner edge 6 of the separation chamber 20, slightly outside of the bay area 25 on the side of the smaller segment of the distribution channel 21. The bag sensor 66 is able to detect red blood cells in a liquid. Each sensor 64, 65, 66 can comprise a photocell including an infra-red LED and a photo-detector.

The separation apparatus further comprises a controller 67 including a microprocessor and a memory for providing the microprocessor with information and programmed instructions relative to the operation of the apparatus. In particular, the microprocessor is programmed for receiving information relative to the various centrifugation speeds at which the rotor is to be rotated during the various stage of a separation process, and information relative to the various transfer flow rates at which separated components are to be transferred from the separation bag 1 into the products bags 2, 3, 4. The information relative to the various transfer flow rates can be expressed, for example, as hydraulic liquid flow rates in the hydraulic circuit, or as rotation speeds of the stepper motor 59 of the hydraulic pumping station 31. The microprocessor is further programmed to receive, directly or through the memory, information from the pressure gauge 63 and from the photocells 64, 65, 66.

The microprocessor is further programmed for controlling the centrifuge motor 40, the stepper motor 59, and the pinch valve members 42, 43, 44 so as to cause the separation apparatus to operate along a predetermined separation protocol.

A first example of separation protocol is as follows.

First stage: the volume of anti-coagulated blood to be separated is transferred into the separation bag before or after the disposable set is loaded in the centrifugation apparatus according to one of the following variants.
- First variant: a volume of anti-coagulated blood to be separated (for example about 500 ml) is transferred into the separation bag before the disposable set is loaded in the centrifugation apparatus. After a clamp has been placed at the connection of each tubes 7, 8, 9 to the separation bag 1, a volume of anti-coagulated blood contained in a collection bag 16 is transferred by gravity in to the separation bag 1. The tube 14 connecting the collection bag 16 to the separation bag is sealed and cut. The separation bag 1 is fitted within the turntable 38, the tubes 7, 8, 9 are engaged in the pinch valve members 42, 44, 43, and the product bags 2, 3, 4, and the secondary bag 10 are placed into the container 37. The pinch valve members 42, 44, 43 are closed and the clamps on the tubes 7, 8, 9 are removed. Alternately, clamps are not placed on tubes 7, 8, 9 when pressure frangible seals are provided in both segments of the distribution channel 21 so as to prevent communication between the product bags 2, 3, 4 and the separation bag 1 as long as the pressure that builds within the separation bag during the operation of the separation apparatus is not high enough to break the frangible seals.
- Second variant: same as the first variant, except that the separation bag 1, which contains a volume of anticoagulant, is directly connected to a donor and the blood of the donor is directly drawn into the separation bag 1, which is thus also used as a collection bag.

Second stage: the air present in the separation bag 1 is purged into the product bag 4 in which the red blood cell component is to be later transferred.

The pinch valve members 42, 43 are closed and the pinch valve member 44 in which the tube 9 is engaged is open. The rotor is set in motion by the centrifuge motor 40 and its rotation speed increases steadily until it rotates at a first, high centrifugation speed (for example, about 3200 RPM). Before the rotor rotates at the first centrifugation speed, the pumping station 31 is actuated so as to pump hydraulic liquid at a constant flow rate (for example, about 240ml/min) into the hydraulic chamber 32 and consequently squeeze the separation bag 1. The air present in the separation bag 1 is expelled into the product bag 4 for the red blood cell component. When the channel sensor 64 detects a liquid in the distribution channel 21, the pumping station 31 is stopped and the pinch valve member 44 is closed.

When the distribution channel 21 of the separation bag 1 is initially closed by frangible seals, the transfer of air from the separation bag 1 into the product bag 4 occurs once the pressure building up in the separation bag 1 is high enough to cause the frangible seals to break.

Note that, alternately, the air contained in the separation bag 1 could be expelled into either the product bag 2 for the plasma component or the product bag 3 for the platelet component. It is however of interest to expel the air in the bag 4 for the red blood cell component because this will allow the red blood cell component to be later transferred by gravity from the product bag 4 into the secondary bag 10.

Third stage: the blood within the separation chamber is sedimented to a desired level.

At the onset of this stage, the three pinch valve members 42, 43 and 44 are closed. The rotor is rotated at the first high centrifugation speed (for example, about 3200 RPM) for a predetermined period of time (for example, about 220 seconds) that is selected so that, whatever the hematocrit of the volume of the blood initially transferred in the separation chamber 1, the blood sediments therein at the end of the selected period to a point where the hematocrit of the outer annular red blood cell layer is about 90 and the inner annular plasma layer plasma does not substantially contain anymore cells, the platelets and the white blood cells occupying then an intermediary annular layer between the red blood cell layer and the plasma layer.

Fourth stage: a first component (plasma component) is transferred into the product bag 2.

At the onset of this stage, the three pinch valve members 42, 43, 44 are closed. Throughout the fourth stage, the rotor is rotated at the first high centrifugation speed (for example, about 3200 RPM). After a predetermined period of time after the bag sensor 66 has stopped detecting red blood cells, which can happen before the end of the predetermined sedimentation period, the pinch valve member 42 controlling the access to the plasma component bag 2 is opened and the pumping station 31 is actuated so as to pump hydraulic liquid at a constant flow rate (for example, about 220ml/min) into the hydraulic chamber 32 and consequently squeeze the separation bag 1 so as to cause the transfer of a first portion of the plasma into the product bag 2, whereas a second portion of the plasma (for example, about 60 ml) remains in the separation bag 1. The pumping station 31 is stopped when a predetermined period of time has lapsed after the bag sensor 66 has detected red blood cells.

The transfer flow rate of the plasma component (which is directly related to the flow rate of the hydraulic fluid) is selected to be as high as possible without disturbing the platelet layer so as to avoid contaminating the plasma component with platelets.

Fifth stage: an Intermediate component (platelet component) is prepared in the separation bag 1.
- First variant: the pinch valve member 43 controlling the access to the platelet component bag 3 is open, and the pinch valve members 42, 44 are closed. The rotation speed of the rotor is rapidly decreased from the first centrifugation speed to a second centrifugation speed (for example, from about 3200 RPM to about 2000 RPM, within about 10 seconds) so as to form an intermediate component resulting from the suspension of the platelets into the second portion of the plasma, whereas the red blood cell layer and the suspended platelet layer remains substantially separated.
- Second variant: the three pinch valve members 42, 43, 44 are closed. The rotation speed of the rotor is rapidly decreased from the first centrifugation speed to a second centrifugation speed (for example, from about 3200 RPM to about 1000 RPM, within about 20 seconds) so as to mix red blood cells, the platelets and the second portion of the plasma. The rotation speed of the rotor is then increased from the second centrifugation speed to a third centrifugation speed, lower that the first centrifugation speed (for example, from about 1000 RPM to about 2500 RPM), so as to separate in the separation bag 1 a red blood cell component and an intermediate component comprising a suspension of platelets in plasma.

Sixth stage: the intermediate component (platelet component) is transferred into the product bag 3.

The pinch valve member 43 controlling the access to the platelet component bag 3 is open and the pinch valve members 42, 44 are closed. The rotor is rotated at the second centrifugation speed (for example, about 2000 RPM, if the preceding stage is the first variant of the fifth stage) or at the third rotation speed (for example, about 2500 RPM, if the preceding stage is the second variant of the fifth stage). The pumping station 31 is actuated so as to pump hydraulic liquid at a first flow rate into the hydraulic chamber 32 and consequently squeeze the separation bag 1 and cause the transfer of the platelet component into the product bag 3. The first flow rate (for example, about 140 ml/min) is substantially lower than the flow rate (for example, about 220 ml/min) at which the plasma component is transferred into the product bag 2 in the fourth stage. The first transfer flow rate of the platelet component (which is directly related to the first flow rate of the hydraulic fluid) is selected to be high enough for preventing the suspended platelets from sedimenting, without at the same time triggering the activation of the platelets.

When the bay sensor 65 detects red blood cells, the pumping station 31 is actuated so as to pump hydraulic liquid into the hydraulic chamber 32 at a second flow rate (for example 40 ml/min) that is substantially lower then the first flow rate, in order to prevent the contamination of the platelet component by red blood cells.

When the hydraulic liquid has been pumped into the hydraulic chamber 32 at the second flow rate for a predetermined period of time (for example, about 4 seconds), the pumping station is actuated so as to pump the hydraulic liquid at a third flow rate (for example, about 20 ml/min) that is lower than the second flow rate, until a predetermined period of time (for example, about 12 seconds) has lapsed after the channel sensor 64 has detected red blood cells. The pumping station 31 is then stopped.

Seventh stage: the third component (red blood cell component) is transferred into the product bag 4.

The pinch valve member 44 controlling the access to the red blood cell component bag 4 is open and the pinch valve members 42, 43 are closed. The rotation speed of the rotor is decreased from the second centrifugation speed (for example, about 2000 RPM) or the third centrifugation speed (for example, about 2500 RPM) to a fourth, lower, centrifugation speed (for example, about 1500 RPM). The pumping station 31 is actuated so as to pump hydraulic liquid at a first flow rate into the hydraulic chamber 32 and consequently squeeze the separation bag 1 so as to cause the transfer of the red blood cell component into the product bag 4. The first flow rate (for example, about 350 ml/min) is substantially higher than the flow rate (for example, about 220 ml/min) at which the plasma component is transferred into the product bag 2 in the fourth stage. The first transfer flow rate of the red blood cell component (which is directly related to the flow rate of the hydraulic fluid) is selected to be as high as possible without damaging the red blood cells (hemolysis).

When the pressure of the hydraulic liquid measured by the pressure gauge 63 reaches a first high pressure threshold (for example, about 0.7 bar), the flow rate of the hydraulic liquid is decreased from the first flow rate to a second flow rate (for example, about 100 ml/min).

When the pressure of the hydraulic liquid measured by the pressure gauge 63 reaches a second high pressure threshold (for example, about 1.6 bar), the flow rate of the hydraulic liquid is further decreased from the second flow rate to a third flow rate (for example, about 37 ml/min).

The second and third transfer flow rates of the red blood cell component (which are directly related to the flow rate of the hydraulic fluid) are selected so that a maximal portion of the red blood cell component is transferred into the red blood cell component bag 4.

When a predetermined period of time (for example, about 30 seconds) has lapsed after the pressure of the hydraulic liquid has reached the second pressure threshold, the rotation speed of the rotor is decreased until the rotor stops, the pumping station 31 is actuated so as to pump the hydraulic liquid from the hydraulic chamber 32 at a high flow rate (for example, about 800 ml/min) until it the hydraulic chamber 32 is empty, and the three pinch valve members 42, 43, 44 are actuated so as to seal and cut the tubes 7, 8, 9.

Any of the at least one first transfer flow rate for the plasma component (one flow rate in the example described above), the at least one second transfer flow rate for the platelet component (three successive flow rates in the example described above), the at least third first transfer flow rate for the red blood cell component (three successive flow rates in the example described above) may be substantially constant, as in the example described above, or it may vary and, for example, comprise a ramp or a series of small steps.

According to a second example of separation protocol, a volume of blood is separated into four components, a plasma component, a first intermediate component (comprising platelets suspended in plasma), a second intermediate component (comprising one of the two types of white blood cells, namely mononuclear cells) and a red blood cell component.

In this second separation protocol, after the stage of transferring the platelet component into a platelet bag, mononuclear cells are transferred into either a specific mononuclear cell bag or into a whole blood collection bag, which, in this case, is not disconnected from the separation bag after the initial stage of transferring the volume of anti-coagulated blood from the collection bag into the separation bag. The transfer of the mononuclear cells occurs at the same rotation speed as the transfer of the platelets (for example, about 2500 RPM), at a transfer flow rate that is higher (for example, about 40ml/mn) than the third and final transfer flow rate of the platelets (for example, about 20ml/mn). The transfer of the mononuclear cell component is completed after a predetermined period of time has lapsed.

According to the second separation protocol, the red blood cells that remain in the separation bag, after the stage of transferring the mononuclear cell component into the mononuclear cell bag, are transferred into a red blood cell bag by gravity, after the centrifuge has stopped and the separation bag has been removed, from the centrifuge. In a set of bags adapted to this second protocol, the red blood cell bag is preferably connected at the outer edge of the separation bag. If such a set of bags is used, the air present in the separation bag at the onset of the separation process is then expelled in one of the component bags connected to the inner edge of the separation bag, for example in the mononuclear cell bag.

According to a third example of separation protocol, a volume of blood is separated into three components, a plasma component, an intermediate component (comprising platelets and mononuclear cells), and a red blood cell component.

When the volume of anti-coagulated blood has sedimented to the desired level, then a major portion of the plasma component is transferred into a plasma bag at a maximal flow rate (for example, about 220ml/mn). The transfer of the plasma is stopped after a determined period of time has lapsed after red blood cells are detected by the bag sensor 66 or by the bay sensor 65 depending on the residual volume of plasma that it is desired to leave in the separation bag. The intermediate component (residual plasma, platelets, mononuclear cells and some red blood cells) is then transferred into an intermediate component bag, which can be either a specific component bag or a whole blood collection bag 16. The transfer of the platelet/mononuclear cell component occurs at the same rotation speed as the transfer of the plasma (for example, about 3200 RPM), also at a constant transfer flow rate, but lower (for example, about 140ml/mn) than the transfer flow rate of the plasma. The transfer of the platelet/mononuclear cell component is completed after a pre-determined volume has been expressed into the intermediate component bag.

In the third separation protocol, the red blood cells that remain in the separation bag, after the stage of transferring the platelet/mononuclear cell component into the intermediate component bag, are transferred into a red blood cell bag by gravity, after the centrifuge has stopped and the separation bag has been removed from the centrifuge.

According to a fourth example of separation protocol, a volume of blood is separated into two components, a plasma component and a cell component (platelets, white blood cells and red blood cells). An appropriate set of separation bags may comprise a separation bag connected to a unique component (plasma) bag.

In this fourth separation protocol the air initially present in the separation bag 1 is expelled in the plasma bag. When the volume of anti-coagulated blood has sedimented to the desired level, then a plasma component is transferred into the plasma bag at two different rates: a major portion of the plasma is transferred at a maximal flow rate and the remaining portion of the plasma is then transferred at a lower flow rate. The second flow rate is selected so that a maximum volume of plasma is transferred into the plasma bag and the contamination of the plasma by the blood cells is avoided. The cell component remains in the separation bag for further processing after the centrifuge has stopped.

It will be apparent to those skilled in the art that various modifications can be made to the apparatus and method described herein. Thus, it should be understood that the invention is not limited to the subject matter discussed in the specification. Rather, the present invention is intended to cover modifications and variations.

## Claims

1. An apparatus for separating a volume of composite liquid into at least a first component, an intermediate component including a second component, and a third component, the volume of composite fluid being contained in a flexible separation bag (1) connected to at least a first component bag (2) and an intermediate component bag (3), the apparatus comprising:
a centrifuge having a rotor (35, 36, 37, 38) for spinning the separation bag (1);
a squeezing member (31, 54, 55) for squeezing the separation bag (1) and causing the transfer of at least a first portion of the first component from the separation bag (1) into the first component bag (2) and the transfer of the intermediate component from the separation bag (1) into the intermediate component bag (3);
a memory for storing at least one centrifugation speed allowing for the sedimentation of the at least first, the second and the third components in the separation bag (1), and information related to at least one first transfer flow rate of the first component into the first component bag (2) and at least one second transfer flow rate of the intermediate component into the intermediate component bag (3), whereby the at least one first transfer flow rate and the at least one second transfer flow rate are different; and
a control unit (67) programmed:
for receiving from the memory the at least one centrifugation speed and the information related to the at least one first transfer flow rate and the at least one second transfer flow rate; and
for causing the rotor (35, 36, 37, 38) to rotate at the at least one centrifugation speed; and
for causing, after sedimentation of the at least first, the second and the third components in the separation bag (1), the squeezing member (31, 54, 55) to squeeze the separation bag (1) so as to transfer the first portion of the first component from the separation bag (1) into the first component bag (2) at the at least one first transfer flow rate, whereby a second portion of the first component remains in the separation bag (1);
for causing, after the transfer of the first portion of the first component into the first component bag (2), a variation of the centrifugation speed so as to mix the second component with the second portion of the first component and form the intermediate component; and
for causing, after the forming of the intermediate component, the squeezing member (31, 54, 55) to squeeze the separation bag (1) so as to transfer the intermediate component from the separation bag (1) into the intermediate component bag (3) at the at least one second transfer flow rate.

2. An apparatus according to claim 1, wherein the at least one second transfer flow rate comprises an initial flow rate and a final flow rate, the final flow rate being lower than the initial flow rate.

3. An apparatus according to claim 2, further comprising:
a first sensor (64) for detecting the third component on a pathway of a fluid to the intermediate component bag (3); and
a second sensor (65) for detecting the third component on a pathway of a fluid to the intermediate component bag (3) upstream of the first sensor (64), wherein the control unit (67) is further programmed for receiving information from the first sensor (64) and the second sensor (65) and for causing the transfer of the intermediate component at the initial flow rate until the first sensor (64) detects the third component and at the final flow rate when the second sensor (65) detects the third component.

4. An apparatus according to claim 1, wherein the control unit (67) is further programmed
for causing the rotor (35, 36, 37, 38) to rotate at a first centrifugation speed during the transfer of the first portion of the first component from the separation bag (1) into the first component bag (2); and
for causing a rapid decrease of the centrifugation speed from the first centrifugation speed to a second centrifugation speed so as to mix the second component with the second portion of the first component and form the intermediate component.

5. An apparatus according to claim 4, further comprising:
a first valve member (42) mounted on the rotor (35, 36, 37, 38) for interacting with a first tube (7) connecting the separation bag (1) to the first component bag (2) and selectively allowing or blocking a flow of fluid therethrough;
a second valve member (43) mounted on the rotor (35, 36, 37, 38) for interacting with a second tube (8) connecting the separation bag (1) to the intermediate component bag (3) and selectively allowing or blocking a flow of fluid therethrough; and
wherein the control unit (67) is further programmed for causing the first valve member (42) to close and the second valve member (43) to open during the mixing of the second component with the second portion of the first component and the formation the intermediate component.

6. An apparatus according to claim 1, further comprising:
a first valve member (42) mounted on the rotor (35, 36, 37, 38) for interacting with a first tube (7) connecting the separation bag (1) to the first component bag (2) and selectively allowing or blocking a flow of fluid therethrough;
a second valve member (43) mounted on the rotor (35, 36, 37, 38) for interacting with a second tube (8) connecting the separation bag (1) to the intermediate component bag (3) and selectively allowing or blocking a flow of fluid therethrough; and
a sensor (66) for detecting the third component on a pathway of a fluid to the first component bag (2), wherein the control unit (67) is further programmed for receiving information from the said sensor (66) and for controlling the first and the second valve members (42, 43).

7. Apparatus according to any one of claims 1 to 6, wherein the control unit (67) is further programmed for causing a transfer of air from the separation bag (1) into one of the component bags before the transfer of the first component from the separation bag (1) into the first component bag (2).

8. An apparatus according to claim 1, wherein
the squeezing member (31, 54, 55) is further for causing the transfer of the third component into a third component bag (4) connected to the separation bag (1);
the memory is further for storing information related to at least one third transfer flow rate of the third component into the third component bag (4), whereby the at least one third transfer flow rate is different from the at least one second transfer flow rate; and
the control unit (67) is further programmed:
for receiving from the memory the information related to the at least one third transfer flow rate; and
for causing the squeezing member (31, 54, 55) to squeeze the separation bag (1) so as to transfer the third component from the separation bag (1) into the third component bag (4) at the at least one third transfer flow rate.

9. An apparatus according to claim 8, wherein, during the transfer of the third component from the separation bag (1) into the third component bag (4), the control unit (67) is programmed for causing the rotor (35, 36, 37, 38) to rotate at a centrifugation speed that is less than the rotation speed at which the rotor (35, 36, 37, 38) rotates during the transfer of the intermediate component into the intermediate component bag (3).

10. An apparatus according to any one of claims 8 and 9, further comprising:
a first valve member (42) mounted on the rotor (35, 36, 37, 38) for interacting with a first tube (7) connecting the separation bag (1) to the first component bag (2) and selectively allowing or blocking a flow of fluid therethrough;
a second valve member (43) mounted on the rotor (35, 36, 37, 38) for interacting with a second tube (8) connecting the separation bag (1) to the intermediate component bag (3) and selectively allowing or blocking a flow of fluid therethrough; and
a third valve member (44) mounted on the rotor (35, 36, 37, 38) for interacting with a third tube (9) connecting the separation bag (1) to the third component bag (4) and selectively allowing or blocking a flow of fluid therethrough, wherein the control unit (67) is further programmed for controlling the first, the second and the third valve members (42, 43, 44).

11. An apparatus according to claim 10, further comprising an empty state sensor (63) for detecting when the separation bag (1) is substantially empty, wherein the control unit (67) is further programmed for receiving information from the empty sate sensor (63) and for causing the rotor (35, 36, 37, 38) to stop rotating after detection by the empty state sensor (63) that the separation bag (1) is substantially empty.

12. An apparatus according to claim 11, wherein the rotor (35, 36, 37, 38) comprises:
a turntable (38) for supporting the separation bag (1), and
a lid (49) that can be secured to the turntable (38) for enclosing the flexible separation bag (1), and wherein the squeezing member (31, 54, 55) comprises:
a flexible diaphragm (54) secured to the turntable (38),
a pumping station (31) for pumping a hydraulic fluid into and out of an expandable chamber (32) delimited between the turntable (38) and the flexible diaphragm (54), whereby the flexible separation bag (1) is being squeezed against the lid (49) when the hydraulic fluid is pumped into the expandable chamber (32); and
a pressure sensor (63) for sensing the pressure of the hydraulic fluid, wherein the empty sate sensor for detecting when the separation bag (1) is substantially empty is the pressure sensor.

13. An apparatus according to claim 12, wherein, during the transfer of the third component from the separation bag (1) into the third component bag (4), the control unit (67) is further programmed for causing the transfer of the third component at a first flow rate until the hydraulic pressure measured by the pressure sensor (63) reaches a determined pressure threshold, and at a second flow rate after the hydraulic pressure measured by the pressure sensor (63) has reached the determined pressure threshold, the second flow rate being lower than the first flow rate.

14. An apparatus according to any one of claims 10 to 13, further comprising a sensor (64) for detecting a fluid on a pathway from the separation bag (1) to the third component bag (4), wherein the control unit (67) is further programmed receiving information from the sensor (64) and for causing a transfer of air from the separation bag (1) into the third component bags by causing:
the first and second valve member (42, 43) to close;
the third valve member (44) to open; and
the squeezing member (31, 54, 55) to squeeze the separation bag (1) until the sensor (64) detects the first component.

15. An apparatus according to any one of claims 1 to 14, wherein the composite liquid comprises whole blood, the first component comprises plasma, the second component comprises platelets, the third component comprise red blood cells, and the intermediate component comprises plasma and platelets.

## Patentansprüche

1. Vorrichtung zum Trennen eines Volumens einer gemischten Flüssigkeit in mindestens eine erste Komponente, eine Zwischenkomponente, beinhaltend eine zweite Komponente, und eine dritte Komponente, wobei das Volumen des gemischten Fluids in einem elastischen Trennbeutel (1) enthalten ist, der mit mindestens einem ersten Komponentenbeutel (2) und einem Zwischenkomponentenbeutel (3) verbunden ist, wobei die Vorrichtung umfasst:
eine Zentrifuge mit einem Rotor (35, 36, 37, 38) zum Schleudern des Trennbeutels (1);
ein Pressteil (31, 54, 55) zum Zusammenpressen des Trennbeutels (1) und zum Veranlassen des Übertragens mindestens eines ersten Teils der ersten Komponente aus dem Trennbeutel (1) in den ersten Komponentenbeutel (2) und des Übertragens der Zwischenkomponente aus dem Trennbeutel (1) in den Zwischenkomponentenbeutel (3);
einen Speicher zum Speichern von mindestens einer Zentrifugalgeschwindigkeit unter Berücksichtigung der Ablagerung der mindestens ersten, der zweiten und der dritten Komponenten im Trennbeutel (1) und von Informationen hinsichtlich mindestens einer ersten Übertragungsflussrate von der ersten Komponente in den ersten Komponentenbeutel (2) und mindestens einer zweiten Übertragungsflussrate von der Zwischenkomponente in den Zwischenkomponentenbeutel (3), wobei die mindestens eine erste Übertragungsflussrate und die mindestens eine zweite Übertragungsflussrate unterschiedlich sind; und
eine Steuereinheit (67), programmiert zum:
Empfangen der mindestens einen Zentrifugalgeschwindigkeit und der Informationen hinsichtlich der mindestens einen ersten Übertragungsflussrate und der mindestens einen zweiten Übertragungsflussrate vom Speicher; und
Veranlassen des Rotors (35, 36, 37, 38) zum Rotieren in der mindestens einen Zentrifugalgeschwindigkeit; und
Veranlassen des Pressteils (31, 54, 55) zum Zusammenpressen des Trennbeutels (1) nach der Ablagerung der mindestens ersten, der zweiten und der dritten Komponenten in den Trennbeutel (1), sodass der erste Teil der ersten Komponente in der mindestens einen ersten Übertragungsflussrate aus dem Trennbeutel (1) in den ersten Komponentenbeutel (2) übertragen wird, wobei ein zweiter Teil der ersten Komponente im Trennbeutel (1) verbleibt;
Veranlassen einer Variation der Zentrifugalgeschwindigkeit nach dem Übertragen des ersten Teils der ersten Komponente in den ersten Komponentenbeutel (2), sodass die zweite Komponente mit dem zweiten Teil der ersten Komponente vermischt wird und die Zwischenkomponente gebildet wird; und
Veranlassen des Pressteils (31, 54, 55) zum Zusammenpressen des Trennbeutels (1) nach dem Bilden der Zwischenkomponente, sodass die Zwischenkomponente in der mindestens einen zweiten Übertragungsflussrate aus dem Trennbeutel (1) in den Zwischenkomponentenbeutel (3) übertragen wird.

2. Vorrichtung nach Anspruch 1, wobei die mindestens eine zweite Übertragungsflussrate eine Ausgangsflussrate und eine Endflussrate umfasst, wobei die Endflussrate niedriger ist als die Ausgangsflussrate.

3. Vorrichtung nach Anspruch 2, des Weiteren umfassend:
einen ersten Sensor (64) zum Erkennen der dritten Komponente auf einem Weg eines Fluids zum Zwischenkomponentenbeutel (3); und
einen zweiten Sensor (65) zum Erkennen der dritten Komponente auf einem Weg eines Fluids zum Zwischenkomponentenbeutel (3) stromaufwärts des ersten Sensors (64), wobei die Steuereinheit (67) des Weiteren dazu programmiert ist, Informationen vom ersten Sensor (64) und vom zweiten Sensor (65) zu empfangen und das Übertragen der Zwischenkomponente in der Ausgangsflussrate zu veranlassen, bis der erste Sensor (64) die dritte Komponente erkennt, und in der Endflussrate, wenn der zweite Sensor (65) die dritte Komponente erkennt.

4. Vorrichtung nach Anspruch 1, wobei die Steuereinheit (67) des Weiteren dazu programmiert ist,
den Rotor (35, 36, 37, 38) dazu zu veranlassen, in einer ersten Zentrifugalgeschwindigkeit während des Übertragens des ersten Teils der ersten Komponente aus dem Trennbeutel (1) in den ersten Komponentenbeutel (2) zu rotieren; und
eine rasche Senkung der Zentrifugalgeschwindigkeit von der ersten Zentrifugalgeschwindigkeit auf eine zweite Zentrifugalgeschwindigkeit zu veranlassen, sodass die zweite Komponente mit dem zweiten Teil der ersten Komponente vermischt wird und eine Zwischenkomponente gebildet wird.

5. Vorrichtung nach Anspruch 4, des Weiteren umfassend:
ein erstes Ventilteil (42), angebracht am Rotor (35, 36, 37, 38), zum Zusammenwirken mit einem ersten Rohr (7), das den Trennbeutel (1) mit dem ersten Komponentenbeutel (2) verbindet, und zum selektiven Ermöglichen oder Verhindern eines Fluidflusses durch dieses;
ein zweites Ventilteil (43), angebracht am Rotor (35, 36, 37, 38), zum Zusammenwirken mit einem zweiten Rohr (8), das den Trennbeutel (1) mit dem Zwischenkomponentenbeutel (3) verbindet, und zum selektiven Ermöglichen oder Verhindern eines Fluidflusses durch dieses; und
wobei die Steuereinheit (67) des Weiteren dazu programmiert ist, das erste Ventilteil (42) dazu zu veranlassen, zu schließen, und das zweite Ventilteil (43) dazu zu veranlassen, zu öffnen, und zwar während des Mischens der zweiten Komponente mit dem zweiten Teil der ersten Komponente und der Bildung der Zwischenkomponente.

6. Vorrichtung nach Anspruch 1, des Weiteren umfassend:
ein erstes Ventilteil (42), angebracht am Rotor (35, 36, 37, 38) zum Zusammenwirken mit einem ersten Rohr (7), das den Trennbeutel (1) mit dem ersten Komponentenbeutel (2) verbindet, und zum selektiven Ermöglichen oder Verhindern eines Fluidflusses durch dieses;
ein zweites Ventilteil (43), angebracht am Rotor (35, 36, 37, 38), zum Zusammenwirken mit einem zweiten Rohr (8), das den Trennbeutel (1) mit dem Zwischenkomponentenbeutel (3) verbindet, und zum selektiven Ermöglichen oder Verhindern eines Fluidflusses durch dieses; und
einen Sensor (66) zum Erkennen der dritten Komponente auf einem Weg eines Fluids zum ersten Komponentenbeutel (2), wobei die Steuereinheit (67) des Weiteren dazu programmiert ist, Informationen vom Sensor (66) zu empfangen und die ersten und die zweiten Ventilteile (42, 43) zu steuern.

7. Vorrichtung nach einem der Ansprüche 1 bis 6, wobei die Steuereinheit (67) des Weiteren dazu programmiert ist, ein Übertragen von Luft aus dem Trennbeutel (1) in einen der Komponentenbeutel zu veranlassen, bevor die erste Komponente aus dem Trennbeutel (1) in den ersten Komponentenbeutel (2) übertragen wird.

8. Vorrichtung nach Anspruch 1, wobei
das Pressteil (31, 54, 55) des Weiteren dazu vorgesehen ist, das Übertragen der dritten Komponente in einen dritten mit dem Trennbeutel (1) verbundenen Komponentenbeutel (4) zu veranlassen;
der Speicher des Weiteren dazu vorgesehen ist, Informationen hinsichtlich mindestens einer dritten Übertragungsflussrate der dritten Komponente in den dritten Komponentenbeutel (4) zu speichern, wobei die mindestens eine dritte Übertragungsflussrate von der mindestens einen zweiten Übertragungsflussrate abweicht; und
die Steuereinheit (67) des Weiteren programmiert ist zum:
Empfangen der Informationen hinsichtlich der mindestens einen dritten Übertragungsflussrate vom Speicher; und
Veranlassen des Pressteils (31, 54, 55) zum Zusammenpressen des Trennbeutels (1), sodass die dritte Komponente in der mindestens einen dritten Übertragungsflussrate aus dem Trennbeutel (1) in den dritten Komponentenbeutel (4) übertragen wird.

9. Vorrichtung nach Anspruch 8, wobei die Steuereinheit (67) während des Übertragens der dritten Komponente aus dem Trennbeutel (1) in den dritten Komponentenbeutel (4) dazu programmiert ist, den Rotor (35, 36, 37, 38) dazu zu veranlassen, in einer Zentrifugalgeschwindigkeit zu rotieren, die langsamer als die Rotationsgeschwindigkeit ist, in der der Rotor (35, 36, 37, 38) während des Übertragens der Zwischenkomponente in den Zwischenkomponentenbeutel (3) rotiert.

10. Vorrichtung nach einem der Ansprüche 8 und 9, des Weiteren umfassend:
ein erstes Ventilteil (42), angebracht am Rotor (35, 36, 37, 38), zum Zusammenwirken mit einem ersten Rohr (7), das den Trennbeutel (1) mit dem ersten Komponentenbeutel (2) verbindet, und zum selektiven Ermöglichen oder Verhindern eines Fluidflusses durch dieses;
ein zweites Ventilteil (43), angebracht am Rotor (35, 36, 37, 38), zum Zusammenwirken mit einem zweiten Rohr (8), das den Trennbeutel (1) mit dem Zwischenkomponentenbeutel (3) verbindet, und zum selektiven Ermöglichen oder Verhindern eines Fluidflusses durch dieses; und
ein drittes Ventilteil (44), angebracht am Rotor (35, 36, 37, 38), zum Zusammenwirken mit einem dritten Rohr (9), das den Trennbeutel (1) mit dem dritten Komponentenbeutel (4) verbindet, und zum selektiven Ermöglichen oder Verhindern eines Fluidflusses durch dieses, wobei die Steuereinheit (67) des Weiteren dazu programmiert ist, die ersten, die zweiten und die dritten Ventilteile (42, 43, 44) zu steuern.

11. Vorrichtung nach Anspruch 10, des Weiteren umfassend einen Leerzustand-Sensor (63) zum Erkennen, wenn der Trennbeutel (1) im Wesentlichen leer ist, wobei die Steuereinheit (67) des Weiteren dazu programmiert ist, Informationen vom Leerzustand-Sensor (63) zu erhalten und den Rotor (35, 36, 37, 38) dazu zu veranlassen, das Rotieren einzustellen, nachdem der Leerzustand-Sensor (63) erkannt hat, dass der Trennbeutel (1) im Wesentlichen leer ist.

12. Vorrichtung nach Anspruch 11, wobei der Rotor (35, 36, 37, 38) umfasst:
eine Drehscheibe (38) zum Tragen des Trennbeutels (1), und
einen Deckel (49), der an die Drehscheibe (38) zum Einschließen des elastischen Trennbeutels (1) befestigt werden kann, und wobei das Pressteil (31, 54, 55) umfasst:
eine an der Drehscheibe (38) befestigte elastische Membran (54),
eine Pumpstation (31) zum Pumpen von Hydraulikfluid in eine erweiterbare Kammer (32), die zwischen der Drehscheibe (38) und der elastischen Membran (54) abgegrenzt wird, und aus dieser heraus, wobei der elastische Trennbeutel (1) gegen den Deckel (49) gepresst wird, wenn das Hydraulikfluid in die erweiterbare Kammer (32) gepumpt wird; und
einen Drucksensor (63) zum Erkennen des Drucks des Hydraulikfluids, wobei der Leerzustand-Sensor zum Erkennen dessen, ob der Trennbeutel (1) im Wesentlichen leer ist, der Drucksensor ist.

13. Vorrichtung nach Anspruch 12, wobei während des Übertragens der dritten Komponente aus dem Trennbeutel (1) in den dritten Komponentenbeutel (4) die Steuereinheit (67) des Weiteren dazu programmiert ist, das Übertragen der dritten Komponente in einer ersten Flussrate zu veranlassen, bis der vom Drucksensor (63) gemessene hydraulische Druck eine vorgegebene Druckschwelle erreicht hat, und in einer zweiten Flussrate, nachdem der vom Drucksensor (63) gemessene hydraulische Druck die vorgegebene Druckschwelle erreicht hat, wobei die zweite Flussrate niedriger als die erste Flussrate ist.

14. Vorrichtung nach einem der Ansprüche 10 bis 13, des Weiteren umfassend einen Sensor (64) zum Erkennen eines Fluids auf einem Weg vom Trennbeutel (1) in den dritten Komponentenbeutel (4), wobei die Steuereinheit (67) des Weiteren dazu programmiert ist, Informationen vom Sensor (64) zu empfangen und ein Übertragen von Luft aus Trennbeutel (1) in die dritten Komponentenbeutel zu veranlassen, und zwar durch das Veranlassen eines:
Schließens des ersten und zweiten Ventilteils (42, 43);
Öffnens des dritten Ventilteils (44); und
Pressens des Trennbeutels (1) durch das Pressteil (31, 54, 55), bis der Sensor (64) die erste Komponente erkennt.

15. Vorrichtung nach einem der Ansprüche 1 bis 14, wobei die gemischte Flüssigkeit Vollblut umfasst, die erste Komponente Plasma umfasst, die zweite Komponente Blutplättchen umfasst, die dritte Komponente rote Blutkörperchen umfasst und die Zwischenkomponente Plasma und Blutplättchen umfasst.

## Revendications

1. Appareil pour séparer un volume de liquide composite en au moins un premier constituant, un constituant intermédiaire comprenant un deuxième constituant, et un troisième constituant, le volume de fluide composite étant contenu dans une poche de séparation souple (1) raccordée à au moins une poche de premier constituant (2) et une poche de constituant intermédiaire (3), l'appareil comprenant :
une centrifuge possédant un rotor (35, 36, 37, 38) pour faire tourner la poche de séparation (1) ;
un élément de compression (31, 54, 55) pour presser la poche de séparation (1) et provoquer le transfert d'au moins une première fraction du premier constituant depuis la poche de séparation (1) dans la poche de premier constituant (2) et le transfert du constituant intermédiaire depuis la poche de séparation (1) dans la poche de constituant intermédiaire (3) ;
une mémoire pour stocker au moins une vitesse de centrifugation permettant la sédimentation de l'au moins premier, du deuxième et du troisième constituant dans la poche de séparation (1), et les informations relatives à au moins un premier débit de transfert du premier constituant dans la poche de premier constituant (2) et au moins un deuxième débit de transfert du constituant intermédiaire dans la poche de constituant intermédiaire (3), moyennant quoi l'au moins un premier débit de transfert et l'au moins un deuxième débit de transfert sont différents ; et
une unité de commande (67) programmée :
pour recevoir de la mémoire l'au moins une vitesse de centrifugation et les informations relatives à l'au moins un premier débit de transfert et l'au moins un deuxième débit de transfert ; et
pour amener le rotor (35, 36, 37, 38) à effectuer une rotation à l'au moins une vitesse de centrifugation ; et
pour amener, après sédimentation des au moins premier, deuxième et troisième constituants dans la poche de séparation (1), l'élément de compression (31, 54, 55) à presser la poche de séparation (1) afin de transférer la première fraction du premier constituant depuis la poche de séparation (1) dans la poche de premier constituant (2) à l'au moins un premier débit de transfert, moyennant quoi une deuxième fraction du premier constituant reste dans la poche de séparation (1) ;
pour provoquer, après le transfert de la première fraction du premier constituant dans la poche de premier constituant (2), une variation de la vitesse de centrifugation afin de mélanger le deuxième constituant avec la deuxième fraction du premier constituant et de former le constituant intermédiaire ; et
pour amener, après la formation du constituant intermédiaire, l'élément de compression (31, 54, 55) à presser la poche de séparation (1) afin de transférer le constituant intermédiaire depuis la poche de séparation (1) dans la poche de constituant intermédiaire (3) à l'au moins un deuxième débit de transfert.

2. Appareil selon la revendication 1, où l'au moins deuxième débit de transfert comprend un débit initial et un débit final, le débit final étant plus bas que le débit initial.

3. Appareil selon la revendication 2, comprenant en outre :
un premier capteur (64) pour détecter le troisième constituant sur un trajet d'un fluide vers la poche de constituant intermédiaire (3) ; et
un deuxième capteur (65) pour détecter le troisième constituant sur un trajet d'un fluide vers la poche de constituant intermédiaire (3) en amont du premier capteur (64), où l'unité de commande (67) est en outre programmée pour recevoir des informations du premier capteur (64) et du deuxième capteur (65) et pour provoquer le transfert du constituant intermédiaire au débit initial jusqu'à ce que le premier capteur (64) détecte le troisième constituant et au débit final lorsque le deuxième capteur (65) détecte le troisième constituant.

4. Appareil selon la revendication 1, où l'unité de commande (67) est en outre programmée
pour amener le rotor (35, 36, 37, 38) à effectuer une rotation à une première vitesse de centrifugation lors du transfert de la première fraction du premier constituant depuis la poche de séparation (1) dans la poche de premier constituant (2) ; et
pour entraîner une diminution rapide de la vitesse de centrifugation de la première vitesse de centrifugation à une deuxième vitesse de centrifugation afin de mélanger le deuxième constituant avec la deuxième fraction du première constituant et de former le constituant intermédiaire.

5. Appareil selon la revendication 4, comprenant en outre :
un premier élément de vanne (42) monté sur le rotor (35, 36, 37, 38) pour interagir avec un premier tube (7) raccordant la poche de séparation (1) à la poche de premier constituant (2) et permettant ou empêchant sélectivement un écoulement de fluide dans celui-ci ;
un deuxième élément de vanne (43) monté sur le rotor (35, 36, 37, 38) pour interagir avec un deuxième tube (8) raccordant la poche de séparation (1) à la poche de constituant intermédiaire (3) et permettant ou empêchant sélectivement un écoulement de fluide dans celui-ci ; et
où l'unité de commande (67) est en outre programmée pour amener le premier élément de vanne (42) à se fermer et le deuxième élément de vanne (43) à s'ouvrir lors du mélange du deuxième constituant avec la deuxième fraction du premier constituant et la formation du constituant intermédiaire.

6. Appareil selon la revendication 1, comprenant en outre :
un premier élément de vanne (42) monté sur le rotor (35, 36, 37,38) pour interagir avec un premier tube (7) raccordant la poche de séparation (1) à la poche de premier constituant (2) et permettant ou empêchant sélectivement un écoulement de fluide dans celui-ci ;
un deuxième élément de vanne (43) monté sur le rotor (35, 36, 37, 38) pour interagir avec un deuxième tube (8) raccordant la poche de séparation (1) à la poche de constituant intermédiaire (3) et permettant ou empêchant sélectivement un écoulement de fluide dans celui-ci ; et
un capteur (66) pour détecter le troisième constituant sur un trajet d'un fluide vers la poche de premier constituant (2), où l'unité de commande (67) est en outre programmée pour recevoir des informations dudit capteur (66) et pour commander le premier et le deuxième élément de vanne (42, 43).

7. Appareil selon l'une quelconque des revendications 1 à 6, où l'unité de commande (67) est en outre programmée pour provoquer un transfert d'air de la poche de séparation (1) dans une des poches de constituant avant le transfert du premier constituant depuis la poche de séparation (1) dans la poche de premier constituant (2).

8. Appareil selon la revendication 1, où
l'élément de compression (31, 54, 55) est en outre destiné à provoquer le transfert du troisième constituant dans une poche de troisième constituant (4), raccordée à la poche de séparation (1) ;
la mémoire est en outre destinée à stocker des informations relatives à au moins un troisième débit de transfert du troisième constituant dans la poche de troisième constituant (4), moyennant quoi l'au moins un troisième débit de transfert est différent de l'au moins un deuxième débit de transfert ; et
l'unité de commande (67) est en outre programmée :
pour recevoir de la mémoire les informations relatives à l'au moins un troisième débit de transfert ; et
pour amener l'élément de compression (31, 54, 55) à presser la poche de séparation (1) afin de transférer le troisième constituant depuis la poche de séparation (1) dans la poche de troisième constituant (4) à l'au moins un troisième débit de transfert.

9. Appareil selon la revendication 8, où, lors du transfert du troisième constituant depuis la poche de séparation (1) dans la poche de troisième constituant (4), l'unité de commande (67) est programmée pour amener le rotor (35, 36, 37, 38) à tourner à une vitesse de centrifugation qui est inférieure à la vitesse de rotation à laquelle tourne le rotor (35, 36, 37, 38) lors du transfert du constituant intermédiaire dans la poche de constituant intermédiaire (3).

10. Appareil selon l'une quelconque des revendications 8 et 9, comprenant en outre :
un premier élément de vanne (42) monté sur le rotor (35, 36, 37, 38) pour interagir avec un premier tube (7) raccordant la poche de séparation (1) à la poche de premier constituant (2) et permettant ou empêchant sélectivement un écoulement de fluide dans celui-ci ;
un deuxième élément de vanne (43) monté sur le rotor (35, 36, 37, 38) pour interagir avec un deuxième tube (8) raccordant la poche de séparation (1) à la poche de constituant intermédiaire (3) et permettant ou empêchant sélectivement un écoulement de fluide dans celui-ci ; et
un troisième élément de vanne (44) monté sur le rotor (35, 36, 37, 38) pour interagir avec un troisième tube (9) raccordant la poche de séparation (1) à la poche de troisième constituant (4) et permettant ou empêchant sélectivement un écoulement de fluide dans celui-ci , où l'unité de commande (67) est en outre programmée pour commander le premier, le deuxième et le troisième élément de vanne (42, 43, 44).

11. Appareil selon la revendication 10, comprenant en outre un capteur d'état vide (63) pour détecter le moment où la poche de séparation (1) est sensiblement vide, où l'unité de commande (67) est en outre programmée pour recevoir des informations du capteur d'état vide (63) et amener le rotor (35, 36, 37, 38) à interrompre sa rotation après détection par le capteur d'état vide (63) du fait que la poche de séparation (1) est sensiblement vide.

12. Appareil selon la revendication 11, où le rotor (35, 36, 37, 38) comprend :
un plateau tournant (38) pour supporter la poche de séparation (1), et
un couvercle (49) qui peut être fixé au plateau tournant (38) pour enfermer la poche de séparation souple (1), et où l'élément de compression (31, 54, 55) comprend :
une membrane souple (54) fixée au plateau tournant (38),
une station de pompage (31) pour pomper un fluide hydraulique vers et hors d'une chambre expansible (32) délimitée entre le plateau tournant (38) et la membrane souple (54), moyennant quoi la poche de séparation souple (1) est pressée contre le couvercle (49) lorsque le fluide hydraulique est pompé dans la chambre expansible (32) ; et
un capteur de pression (63) pour détecter la pression du fluide hydraulique, où le capteur d'état vide pour détecter le moment où la poche de séparation (1) est sensiblement vide est le capteur de pression.

13. Appareil selon la revendication 12, où, lors du transfert du troisième constituant depuis la poche de séparation (1) dans la poche de troisième constituant (4), l'unité de commande (67) est en outre programmée pour provoquer le transfert du troisième constituant à un premier débit jusqu'à ce que la pression hydraulique mesurée par le capteur de pression (63) atteigne un seuil de pression déterminé, et à un deuxième débit après que la pression hydraulique mesurée par le capteur de pression (63) a atteint le seuil de pression déterminé, le deuxième débit étant inférieur au premier débit.

14. Appareil selon l'une quelconque des revendications 10 à 13, comprenant en outre un capteur (64) pour détecter un fluide sur un trajet depuis la poche de séparation (1) vers la poche de troisième constituant (4), où l'unité de commande (67) est en outre programmée pour recevoir des informations du capteur (64) et pour provoquer un transfert d'air depuis la poche de séparation (1) dans les poches de troisième constituant en amenant :
le premier et le deuxième élément de vanne (42, 43) à se fermer ;
le troisième élément de vanne (44) à s'ouvrir ; et
l'élément de compression (31, 54, 55) à presser la poche de séparation (1) jusqu'à ce que le capteur (64) détecte le premier constituant.

15. Appareil selon l'une quelconque des revendications 1 à 14, où le liquide composite comprend du sang total, le premier constituant comprend du plasma, le deuxième constituant comprend des plaquettes, le troisième constituant comprend des globules rouges et le constituant intermédiaire comprend du plasma et des plaquettes.
